# EUROPEAN PATENT APPLICATION

(11) **EP 4 553 230 A1**
(43) Date of publication of application: **14.05.2025**
(21) Application number: 23209120.7
(22) Date of filing: 10.11.2023
(51) Int. Cl.: E02D 1/02, E21B 49/00, G01N 29/07

(54) **DEVICE, SYSTEM AND METHOD FOR INVESTIGATING SOIL**

(71) Applicant: ETH Zurich, 8092 Zürich (CH)
(72) Inventor: Anastasopoulos, Ioannis, 8093 Zürich (CH); Alber, Simone, 8093 Zürich (CH); Adamidis, Orestis, 8093 Zürich (CH); Jones, Liam Alexander, 8093 Zürich (CH); Kieper, Andreas, 8093 Zürich (CH); Giger, Jürg, 8093 Zürich (CH); Hagenbuch, Heinrich, 6030 Ebikon (CH)
(74) Representative: Schulz Junghans Patentanwälte PartGmbB

(57) **Abstract**

The invention relates to a device (1) for investigating soil (2), comprising movement elements (10) for propulsion of the device (1) in the soil (2), wherein the device (1) is configured to determine a quantity of at least one physical parameter which is indicative of a force acting on at least one of the movement elements (10) when the respective movement element (10) moves in the soil (2).

The invention also relates to a system (20) for investigating soil (2) as well as a method for investigating soil (2).

## Description

The present invention relates to a device, a system and a method for investigating soil.

The evaluation of soil properties is a crucial task in geotechnical engineering and can be facilitated by means of field and laboratory testing. Field or in-situ testing has a number of advantages over laboratory testing, the most important being that no soil sampling is needed, and therefore soil disturbance can be minimized. However, field-testing requires a probe with sufficient instrumentation, which in turn must be driven into the soil to a certain depth. The deployment of the machinery required to perform the penetration of the probe is expensive and time-consuming. Furthermore, in-situ tests provide only limited local data, which cannot be generalized for the entire geotechnical site due to the spatial variability of soil characteristics. Cost and time often prohibit multi-site testing, which forces practitioners to interpolate values of geotechnical parameters between sparse testing points, increasing the uncertainty in geotechnical design. A common example which illustrates the trade-off between cost of exploration and cost of uncertainty in design is offshore geotechnical testing, where massive, highly specialized vessels are required for the positioning of the probe drilling rig, which is known to be costly. Even worse, with the rapid growth of offshore wind energy, there is currently a shortage of such vessels.

To tackle this problem, prior art document US7055625B1 proposes an autonomous subsurface drilling device for the investigation of soil. The drilling device comprises spaced-apart forward and rearward feet-sections that operate using an inchworm method of mobility with a drill head mounted on least the forward section of the device. The device has an on-board depository for cuttings or core samples, so that they do not have to be passed to the surface through management of a tether tube while the device is in operation deep below the surface. The samples are taken by collecting soil laterally surrounding the drilling device. However, to investigate soil, samples have to be taken and analyzed serially and repeatedly, which takes time and thus reduces the efficiency of the device. Furthermore, the properties of the soil samples taken from the sides can be disturbed by the penetration of the drilling device and the sampling process, such that the properties of the soil determined in that way can be of increased uncertainty.

Based on this, it is subject of the present invention to provide a device, a system and a method for investigating soil, which allows for a more time- and cost-efficient and more reliable investigation of soil compared to the prior art.

This task is solved by a device for investigating soil with the features of claim 1, a system for investigating soil with the features of claim 13 as well as a method for investigating soil with the features of claim 14.

Advantageous embodiments of the invention are given in the corresponding dependent claims and described in the following.

A first aspect of the invention relates to a device for investigating soil. The device comprises movement elements for propulsion of the device in the soil, wherein the device is configured to determine a quantity of at least one physical parameter, which is indicative of a force acting on at least one of the movement elements when the respective movement element moves in the soil.

The term 'soil' according to the present invention relates to soil beneath the surface of a planet, such as the planet earth. For example, the soil can be arranged below seabed, wherein seabed forms a bottom surface of a water reservoir such as sea, an ocean or a lake, covering the seabed. As such, the invention may be for instance applied to investigate soil properties at sites and evaluate the suitability of possible sites for offshore installations, for example for wind energy infrastructure.

The term 'quantity' according to the present invention refers to a magnitude of the respective determined physical parameter.

The at least one physical parameter may be recorded, that is, the physical parameter is determined and saved, for example on a non-transitory storage medium.

In an embodiment, the device is configured to generate a driving force for causing the movement of the respective movement element in the soil, wherein the physical parameter is the driving force acting on the respective movement element. As such, the force acting on the respective movement element can be caused by said driving force. As the respective movement element is moved by the driving force, it experiences a counterforce by the surrounding soil contacting the respective movement element, according to Newton's third law. The driving force required to accomplish the movement of the respective movement element is therefore indicative for the counterforce exerted by the soil, which allows to investigate the soil. For example, harder or stiffer soil is characterized by a larger driving force required to accomplish a unit of displacement of the respective movement element compared to less hard or less stiff soil. The driving force can thus be a physical parameter indicative of the force acting on the respective movement element, when the respective movement element moves in the soil. For example, multiple or all movement elements of the device are configured to be moved by a respective driving force, such that respective driving forces are indicative of respective forces acting on the respective movement element.

According to an embodiment, the device comprises two axial movement elements configured to be moved with respect to each other along a longitudinal axis of the device. The two axial movement elements are also referred to as a first axial movement element and a second axial movement element herein.

In an embodiment, the device comprises a first radial movement device and a second radial movement device. The first radial movement device is arranged at one of the axial movement elements and the second radial movement device is arranged at the other of the two axial movement elements, such that the first radial movement device and the second radial movement device can be moved with respect to each other.

The first radial movement device and the second radial movement device can each comprise at least one radial movement element configured to be moved radially outwards against the soil by means of a respective driving force. The term 'radially outwards' is to be understood with respect to said longitudinal axis of the device.

In another embodiment, the device comprises at least one axial movement device for extending the device along its longitudinal axis by increasing an offset between the two axial movement elements. To this end, the axial movement device can comprise an actuator, for example a hydraulic actuator.

For example, the axial movement device is arranged in a central section between the first radial movement device and the second radial movement device, such that the device can be extended by extending this section, while the spatial extent of a first section comprising the first radial movement device and the spatial extent of a second section comprising the second radial movement device remain constant.

Alternatively, the two axial movement elements each comprise a respective axial movement device, such that the two axial movement elements, and optionally the first radial movement device and the second radial movement device, can be propulsed by their respective axial movement device, which likewise allows for a propulsion of the device in the soil.

According to an embodiment, the movement of at least one of the radial movement elements and/or the axial movement element is configured to be at least partially reversed.

In an embodiment, the device is configured to move through the soil by, optionally repeatedly, moving radially outwards against the soil the radial movement element of the first radial movement device, extending the offset between the radial movement elements of the first radial movement device and the second radial movement device using the axial movement device, moving radially outwards against the soil the radial movement element of the second radial movement device, reversing the movement of the radial movement element of the first radial movement device, and at least partially reversing the extension of the offset between the radial movement elements of the first radial movement device and the second radial movement device using the axial movement device. In this fashion, the device may be propulsed through the soil according to an inchworm-like movement mechanism. As such, the radial movement elements enable a propulsion of the device caused by the axial movement device by acting as anchors of the device in the soil. At the same time, as the device is propulsed through the soil, it is configured to determine the driving forces required to move the movement elements, for example the radial movement elements and the axial movement elements, and thus to determine the corresponding counterforces exerted by the soil, which, optionally in combination with the determination of the respective displacement of the movement elements, allows for a quantification of the properties, such as stiffness and strength, of the surrounding soil. As such, the movement elements, for example the radial movement elements and the axial movement elements, provide both a propulsion of the device through the soil and a tool to determine said quantity of the at least one physical parameter which is indicative of a force acting on at least one of the movement elements when the respective movement element moves in the soil. By combining the movement mechanism with the determination of the quantity of said physical parameter indicative of the force acting on the movement elements, the investigation of soil by the device is substantially speeded up, while delivering reliable information about the soil properties.

The movement mechanism according to the preceding embodiment can be executed repeatedly, wherein the device is propulsed further in the soil using its movement elements, for example the radial movement elements and the axial movement elements. The soil may thus be investigated in situ, under propulsion of the device through the soil.

In an embodiment, the device comprises a pressure-sensitive element, wherein the physical parameter is the force acting on the pressure-sensitive element when the pressure-sensitive element is propulsed in the soil. For example, the pressure-sensitive element can be arranged on a tip or end face of an axial movement element of the device along its longitudinal axis. The pressure-sensitive element can alternatively or additionally be arranged on a surface of at least one radial movement element for contacting the surrounding soil. Each tip or end face of the device along its longitudinal axis can form a penetration zone, where the soil is minimally disturbed. This provides the opportunity to pressure-sensitive elements for the measurement of soil properties in almost undisturbed soil conditions.

For example, at least one pressure-sensitive element is arranged at an end face of at least one of the axial movement elements, which form end faces of the device along its longitudinal axis. As such, as the device is propulsed into the soil with its axial movement elements, the pressure-sensitive element experiences a counterforce of the soil against which it is moved. The counterforce creates a mechanical pressure determined by the pressure-sensitive element, which in turn allows to draw conclusions about the counterforce and thus properties of the soil. Investigating the soil using the determined pressure of the pressure-sensitive element arranged at an end face is particularly advantageous as the pressure determined in this way represents the forces exerted by pristine soil under natural conditions.

According to another embodiment, the device comprises a shear wave emitter and a shear wave detector, wherein the shear wave emitter is configured to emit a shear wave into the soil and wherein the shear wave detector is configured to detect the arrival of the shear wave travelling through the soil. Preferably, the shear wave emitter and the shear wave detector are fixedly attached to the device, such that the shear wave emitter and the shear wave detector remain at a fixed distance with respect to each other, also in case of movements of the device and/or individual movement elements. From a time between an emission event of a shear wave at the shear wave emitter and a detection event of the shear wave at the shear wave detector, the shear wave velocity of the soil between the shear wave emitter and the shear wave detector can be determined, which allows to draw conclusions about the properties of the soil in the vicinity of the device. For example, the shear wave emitter and the shear wave detector are arranged at an end face of at least one moving element forming an end face of the device, where the soil is almost undisturbed. For example, the shear wave emitter is configured to repeatedly emit shear waves into the soil, for example during or upon a movement of the movement element in the soil, and the shear wave detector is configured to repeatedly detect the arrival of shear waves travelling through the soil. This allows to probe the properties of the soil repeatedly and for different positions of the device in the soil.

In yet another embodiment, the device comprises a screw conveyor extending along the longitudinal axis of the device, and wherein the device comprises a rotary actuator for causing a rotational motion of the screw conveyor around the longitudinal axis, such that soil can be conveyed by the screw conveyor and that the device can be moved through the soil under rotation of the screw conveyor. The screw conveyor can comprise or be connected to a cutting edge arranged at the end face of at least one of the axial movement elements, which form end faces of the device along its longitudinal axis. This cutting edge can be engaged during the penetration of the device in the soil, in this way it facilitates the penetration and enables the minimization of the disturbance on the surrounding soil.

According to another embodiment, the first radial movement device comprises a plurality of radial movement elements, for example four radial movement elements, and/or wherein the second radial movement device comprises a plurality of radial movement elements, for example four radial movement elements, wherein the radial movement elements are arranged equidistantly around the longitudinal axis.

In an embodiment, at least one of the movement elements, for example one of the radial movement elements and/or the axial movement elements, is mechanically connected to an actuator for carrying out the movement of the movement elements, for example of the radial movement elements and/or the axial movement elements, in the soil. For example, the actuator is a hydraulic actuator.

In an embodiment, the actuator is a hydraulic actuator and the driving force for causing the movement of the respective movement element in the soil depends on a hydraulic pressure generated by the hydraulic actuator. In this case, the device may comprise a hydraulic pressure sensor configured to determine the hydraulic pressure generated by the hydraulic actuator as the physical parameter.

In this way, the force acting on the movement elements, for example the radial movement elements and/or the axial movement elements can be determined from the pressure generated by the hydraulic actuator.

According to another embodiment, the device is configured to determine a force, a displacement and/or a time associated with the movement of the at least one of the movement elements in the soil. The force, displacement and/or time associated with the movement of the respective movement element in the soil can be physical parameters indicative of the force acting on the respective movement element, when the respective movement element moves in the soil.

The device may be configured to determine a force and a displacement associated with the movement of the at least one movement element in the soil. The driving force necessary to overcome the counterforce can be the physical parameter which is indicative of the force acting on the movement elements. The displacement can be understood as a relative displacement of the movement element with respect to the device under action of the driving force. From the relation of the force and the associated displacement, conclusions regarding the properties of the soil can be drawn. For example, said hydraulic actuator may be used to cause the movement of the respective movement element in the soil, wherein the hydraulic pressure of the hydraulic liquid, for example oil, that is required for the movement, such as said displacement, is indicative of the force acting on the respective movement element.

According to another embodiment, the device comprises a device control unit configured to exchange signals between the device and an external unit. For example, the signals relate to or encode the movement of the device in the soil, such as a maximum depth of soil below sea bed to be investigated by the device. The signals can comprise data concerning a determined quantity of the at least one physical parameter which is indicative of a force acting on the at least one movement element when the respective movement element moves in the soil. For example, the data relate to force values as a function of time and/or location, representing the driving force of an actuator of the device required to move the at least one movement element against the soil, which represents the counterforce exerted by the soil on the movement element, thereby allowing to quantify the properties of the soil, while the device is propulsed into the soil by means of the movement elements.

A second aspect of the invention relates to a system for investigating soil. The system for investigating soil comprises at least one device for investigating soil according to the first aspect of the invention, as well as a deployment unit for positioning the device on a soil surface, such that soil below the soil surface can be investigated using the device.

The soil surface can form an interface between the soil and a surrounding medium. For example, the medium is gas, for instance gas of an atmosphere, for example the atmosphere of the planet earth or the medium is a liquid, for example water. In the latter case, the soil surface can be the seabed.

The deployment unit may comprise a control unit configured to send output signals to the device, particularly the device control unit, and to receive input signals from the device. For example, the output signals sent to the device relate to and control the movement of the device in the soil. The input signals can comprise data concerning said determined quantity of at least one physical parameter indicative of a force acting on the at least one movement element when the respective movement element moves in the soil. To exchange the input and the output signals between the device and the deployment unit, the device and the deployment unit may communicate via a cable connection or a wireless connection.

The deployment unit may comprise an energy supply unit configured to supply the device with energy required for the propulsion of the device by means of its movement elements. For example, the energy supply unit comprises a hydraulic system with a hydraulic liquid reservoir and a hydraulic pump for realizing the movement of the movement elements of the device. To this end, the deployment unit and its hydraulic system can be connected by hydraulic tubes for conducting the hydraulic liquid to hydraulic actuators of the device that realize the movement of the movement elements.

A third aspect of the invention relates to a method for investigating soil, wherein at least one device for investigating soil according to the first aspect of the invention or at least one system for investigating soil according to the second aspect of the invention is positioned on the soil and wherein the movement elements of the device of the system propulse the device in the soil, and at least one quantity of a physical parameter which is indicative of a force acting on at least one of the movement elements when the respective movement element moves in the soil is determined.

In an embodiment, the device or the system is positioned on the soil surface and the device inserts or is inserted into the soil, and wherein soil below the soil surface is investigated using the device.

Particularly, the embodiments of one of the three aspects of the inventions are applicable to another of the three aspects of the invention.

Exemplary embodiments are described below in conjunction with the Figures. The Figures are appended to the claims and are accompanied by text explaining individual features of the shown embodiments and aspects of the present invention. Each individual feature shown in the Figures and/or mentioned in the text of the Figures may be incorporated (also in an isolated fashion) into a claim relating to the first, second and/or the third aspect according to the present invention.
- Fig. 1: shows an embodiment of a device for investigating soil according to the invention;
- Fig. 2: shows an axial movement device of a device for investigating soil according to an embodiment of the invention;
- Fig. 3: shows an embodiment of a device for investigating soil according to the invention, comprising a screw conveyor; and
- Fig. 4: shows an embodiment of a system and a method for investigating soil according to the invention.

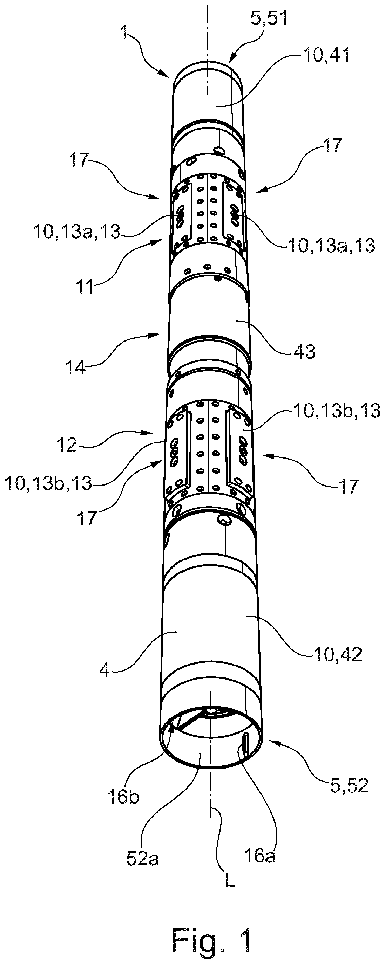

Fig. 1 shows an embodiment of a device 1 for investigating soil 2 according to the invention. The device 1 extends around a longitudinal axis L of the device 1 and is delimited along the longitudinal axis L by two end faces 5, wherein a first end face 51 delimits the device 1 at a first side and wherein a second end face 52 delimits the device 1 at a second side, opposite of the first side.

The device 1 according to the present embodiment comprises a first radial movement device 11 and a second radial movement device 12 that are offset from each other along the longitudinal axis L of the device 1. In this embodiment, the first radial movement device 11 comprises four first radial movement elements 13a and the second radial movement device 12 comprises four second radial movement elements 13b as movement elements 10, that are arranged equidistantly around the longitudinal axis L. The first and second radial movement elements 13a, 13b are configured to be moved radially, that is, perpendicularly to the longitudinal axis L, against soil 2 surrounding the device 1.

In Fig. 1, the first radial movement elements 13a of the first radial movement device 11 are shown in a retracted position and not extended, wherein the outer surfaces 17 of the first radial movement elements 13a of the first radial movement device 11 are essentially flush with the hollow cylinder 4. In contrast, the second radial movement elements 13b of the second radial movement device 12 are shown in an extended position, wherein outer surfaces 17 of the second radial movement elements 13b of the second radial movement device 12 protrude away from a hollow cylinder 4 that delimits the device 1 circumferentially around the longitudinal axis L. To move the first and the second radial movement elements 13a, 13b radially against the soil 2 and away from the longitudinal axis L, the device 1 is configured to generate a driving force applied to one or more of the first and/or the second radial movement elements 13a, 13b that is or are to be moved. To this end, one first or second radial movement element 13a, 13b or more first and/or second radial movement elements 13a, 13b may be mechanically connected to an actuator 15, for example a hydraulic actuator. As the first or second radial movement element 13a, 13b is moved by the driving force, it experiences a counterforce by the surrounding soil 2 contacting the first or second radial movement element 13a, 13b with its outer surface 17, according to Newton's third law. The driving force required to accomplish the movement of the first or second radial movement element 13a, 13b is therefore indicative for the counterforce exerted by the soil 2, which allows to investigate and quantify the properties of the soil 2. The movement of the first and/or second radial movement element 13a, 13b can be determined by a sensor configured to detect the displacement of the outer surface 17 with respect to the hollow cylinder 4, which allows to investigate and quantify the properties of the soil 2. For example, harder or stiffer soils 2 are characterized by a larger driving force required to accomplish a unit of displacement of the outer surface 17 with respect to the longitudinal axis L compared to less hard or less stiff soils 2. The extension of the first and/or the second radial movement element 13a, 13b can be reversed, such that it returns from the extended position to the retracted position. To this end, the first radial movement device 11 and/or the second radial movement device 12 can comprise biasing means, such as springs, that are biased when the first and/or the second radial movement elements 13a, 13b are moved from the retracted to the extended position. As such, the first and/or the second radial movement elements 13a, 13b may be moved back to their retracted positions by releasing energy stored in the biasing means. Alternatively, the movement from the retracted to the extended position may be realized by means of said actuators 15.

The outer surfaces 17 of the first and/or the second radial movement elements 13a, 13b can comprise asperities, and/or ridges with maximum extensions perpendicular to the longitudinal axis L, for example wherein the maximum extensions are within 0.1 mm to 2 mm, particularly within 0.1 mm to 1 mm, more particularly within 0.1 mm to 0.5 mm. As such, the outer surface 17 enhances the friction between the soil 2 and the first or the second radial movement elements 13a, 13b, and thus improves the mechanical anchoring of the first or the second radial movement elements 13a, 13b in the soil 2.

The device 1 shown in Fig. 1 further comprises a first axial movement element 41 and a second axial movement element 42 that form opposite sections of the device 1 along its longitudinal axis L. The first radial movement device 11 is arranged on the first axial movement element 41 and the second radial movement device 12 is arranged on the second axial movement element 42.

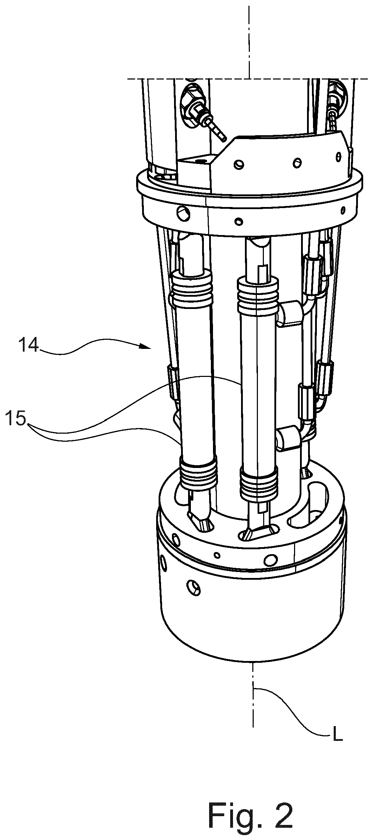

As can further be seen in Fig. 1, the device 1 comprises an axial movement device 14 arranged at a central section 43 between the first axial movement element 41 and the second axial movement element 42, and thus between the first radial movement device 11 and the second radial movement device 12. The axial movement device 14 is configured to extend the device 1 along its longitudinal axis L by extending the central section 43, and thereby increasing the offset between the first radial movement elements 13a of the first radial movement device 11 and the second radial movement elements 13b of the second radial movement device 12. To this end, the axial movement device 14 comprises at least one actuator 15, such as at least one hydraulic actuator, as shown in more detail in Fig. 2, which extends along the longitudinal axis L so as to increase the offset between the first axial movement 41 and the second axial movement element 42, while it is also equipped with sensors measuring the axial driving force and the axial displacement of the axial movement device 14. As the axial movement device 14 extends the central section 43, it propulses the axial movement elements 41,42 into minimally disturbed, natural soil 2, such that a driving force for actuating the axial movement elements 41,42, in conjunction with the measured axial displacement of the axial movement elements 41 delivers particularly reliable information about properties of the soil 2.

The device 1 according to the embodiment shown in Fig. 1 is configured to move through the soil 2 in an inchworm-like fashion according to the following movement mechanism: In a first step, the first radial movement elements 13a of the first radial movement device 11 are moved radially outwards against the soil 2, such that the first axial movement element 41 is anchored in the soil 2. Then, in a second step, the axial movement device 14 extends said a central section 43 of the device 1 arranged between the first axial movement element 41 and the second axial movement element 42 along the longitudinal axis L and thereby increases the offset between the first axial movement element 41 and the second axial movement element 42 and thus the offset between the first radial movement device 11 and the second radial movement device 12 with their respective radial movement elements 13a, 13b. As the central section 43 is extended, the device 1, particularly the second axial movement element 42, is propulsed downward in the drawing plane shown in Fig. 1. Subsequently, in a third step, the second radial movement elements 13b of the second radial movement device 12 are moved radially outwards against the soil 2, such that the second axial movement element 42 of the device 1 is anchored in the soil 2 and the first radial movement elements 13a of the first radial movement device 11 are moved back radially inwardly into their retracted positions. Then, in a fourth step, the extension of the central section 43 is at least partially reversed, whereby the central section 43 and thus the device 1 shortens along the longitudinal axis L and the first axial movement element 41 is pulled towards the second axial movement element 42. The reversal of the extension of the central section 43 by means of the axial movement device 14 can for example be realized by biasing means comprised by the axial movement device 14, as explained for the radial movement devices 11,12 and their radial movement elements 13a, 13b. Alternatively or additionally, the reversal of the extension of the central section 43 can be achieved by the actuator 15.

As such, the first and the second radial movement elements 13a, 13b enable a propulsion of the device 1 caused by the axial movement device 14 by acting as anchors of the device 1 in the soil 2. At the same time, as the device 1 is propulsed through the soil 2, it is configured to determine the driving forces required to move the first and the second radial movement elements 13a,13b and the axial movement elements 41,42, and thus the corresponding counterforces exerted by the soil 2, as well as the corresponding radial displacements of the first and/or the second radial movement elements 13a, 13b and the axial displacements of the axial movement elements 41,42, which allows to draw conclusions and quantify the properties of the surrounding soil 2. As such, the movement elements 10, for example the first and the second radial movement elements 13a, 13b and the axial movement elements 41,41, provide both a protrusion of the device 1 through the soil 2 and a tool to determine a of a physical parameter which is indicative of forces acting on the movement elements 10.

The process explained above may be executed repeatedly, wherein the device 1 is propulsed further in the soil 2 using its movement elements 10, for example the first and the second radial movement elements 13a, 13b and the axial movement elements 41,42. The soil 2 may thus be investigated in situ, under propulsion of the device 1 through the soil 2.

By exchanging the order of the extension and retraction of the first and the second radial movement elements 13a, 13b of the first radial movement device 11 and the second radial movement device 12, the device 1 can be propulsed in the opposite direction and thus upward in the drawing plane shown in Fig. 1.

A direction of propulsion of the device 1 through the soil 2 can be steered by selectively extending and retracting selected first and/or second radial movement elements 13a,13b. For example, if only the two first radial movement elements 13a of the first radial movement device 11 and/or the two second radial movement elements 13b of the second radial movement device 12 visible and indicated in Fig. 1 are repeatedly extended and retracted and the other first and/or second radial movement elements 13a,13b arranged on the other side of the longitudinal axis L remain retracted during the propulsion of the device 1, the device 1 is steered towards a direction oriented out of the drawing plane of Fig. 1.

As can also been seen in Fig. 1, the device 1 comprises a shear wave emitter 16a and a shear wave detector 16b, arranged opposite of each other on an inner surface 52a of the second end face 52 of the device 1. As such, after the second section 42 is propulsed into the soil 2, according to the second step of the movement mechanism explained above, the shear wave emitter 16a emits a shear wave into the soil 2 in between the shear wave emitter 16a and the shear wave detector 16b, which is detected by the shear wave detector 16b. A measured time between an emission event of a shear wave at the shear wave emitter 16a and a detection event of the shear wave at the shear wave detector 16b, in conjunction with the known distance between the shear wave emitter 16a and the shear wave detector 16b is used to measure the shear wave velocity and thus derive the small-strain stiffness of the soil 2 between the shear wave emitter 16a and the shear wave detector 16b. Investigating the soil 2 using the shear wave emitter 16a and the shear wave detector 16b arranged at the second end face 52 is particularly advantageous as the measured shear wave velocity is determined in the minimally disturbed soil 2.

Fig. 2 shows an axial movement device 14 of a device 1 for investigating soil 2 according to an embodiment of the invention. The axial movement device 14 comprises two actuators 15. Two more actuators 15 may be arranged on the opposite side of the longitudinal axis L. The actuators 15 can be hydraulic actuators. The actuators 15 are arranged parallel to the longitudinal axis L of the device 1 and configured to extend parallel to the longitudinal axis L under a driving force. As the actuators 15 extend, the offset between the first axial movement element 41 and the second axial movement element 42, and between the first radial movement device 11 and the second radial movement device 12, is increased, which allows for a propulsion of the device 1 in the soil 2.

For example, to this end, the axial movement device 14 and its actuators 15 are arranged in a central section 43 between the first axial movement element 41 and the second axial movement element 42, and between the first radial movement device 11 and the second radial movement device 12, such that the device 1 can be extended by extending the third section 43, while the spatial extent of the first section 41 and the second section 42 remain constant, as described in the embodiment of Fig. 1.

Alternatively, the first axial movement element 41 and the second axial movement element 42 each comprise a respective axial movement device 14, such that the first axial movement element 41 and the second axial movement element 42, as well as the first radial movement device 11 and the second radial movement device 12, can be propulsed by their respective axial movement devices 14, which likewise allows for a propulsion of the device 1 through the soil 2 by means of its movement elements 10, for example the first and/or the second radial movement elements 13a, 13b and the axial movement elements 41,42.

The axial movement device 14 can be surrounded by two concentric cylinders configured to slide on top of each other when the actuators 15 extend or retract, to avoid that the actuators 15 are exposed to soil 2.

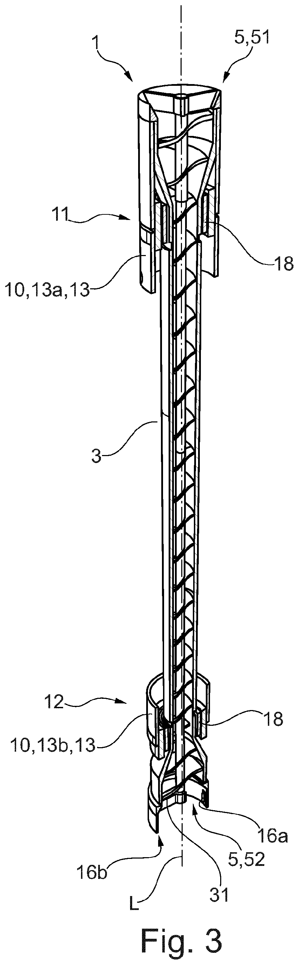

Fig. 3 shows a device 1 for investigating soil 2 according to an embodiment of the invention, comprising a screw conveyor 3. The screw conveyor 3 extends along the longitudinal axis L of the device 1 between its first end face 51 and its second end face 52 and is configured to be set into rotation around the longitudinal axis L by means of two rotary actuators 18 of the device 1, for example two electric rotary actuators, such that soil 2 can be conveyed along the longitudinal axis L. The skilled person will appreciate that a single rotary actuator 18 may suffice for this purpose and that the one or more rotary actuator 18 may be arranged at different positions along the longitudinal axis L compared to the exemplary embodiment of Fig. 3. At least at the second end 52 of the device 1, the screw conveyor is terminated by a cutting edge 31 for cutting soil 2, as the device 1 is propulsed in the soil 2 by its movement elements 10. In this way, during penetration the cutting edge 31 facilitates the process and enables the minimization of the disturbance of the surrounding soil.

Fig. 3 also shows a shear wave emitter 16a and a shear wave detector 16b arranged opposite of each other at the second end 52 of the device 1, which is displaced along the longitudinal axis L with respect to the cutting edge 31 of the screw conveyor 3. The end faces 51, 52 of the device 1 are sharp edged cylinders, which in conjunction with the cutting edge 31 minimize the disturbance of the soil 2 while penetrating. Consequently, the shear wave emitter 16a and the shear wave detector 16b are exposed to minimally disturbed , natural soil 2 as it is propulsed into the soil 2 by the movement elements 10 of the device 1, before the screw conveyor 3 and its cutting edge 31 interact with the soil 2. As such, the screw conveyor 3 does advantageously not affect the properties of the soil between the shear wave emitter 16a and the shear wave detector 16b .

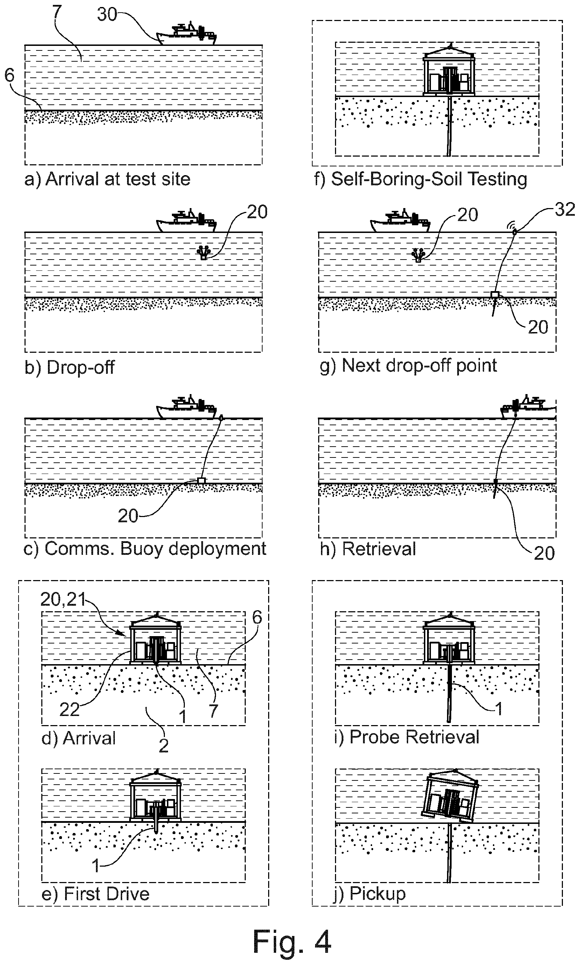

Fig. 4 shows an embodiment of a system 20 and a method for investigating soil 2 according to the invention. As can be seen in the sequence in the left of the two columns in Fig. 4, a watercraft 30 such as a ship or a boat deploys a system 20 according to the invention in a water reservoir 7, for example in the sea, an ocean or a lake, as indicated in section (a) of Fig. 4. The system 20 for investigating soil 2 comprises at least one device 1 for investigating soil 2 according to the invention, as well as a deployment unit 21.

After deployment into the water reservoir 7, the system 20 moves down to seabed 6, which forms a bottom surface of the water reservoir 7, cf. sections (b) and (c) in Fig. 4. As shown in section (d) in Fig. 4, the deployment unit 21 of the system 20 comprises a frame 22 for positioning the device 1 on the seabed 6. Next, as shown in section (f) in Fig. 4, the device 1 enters the soil 2 below the seabed 6. The device 1 may either autonomously enter the soil 2 using its movement elements 10, or it may be inserted into the soil 1 by means of the deployment unit 21 and a corresponding insertion mechanism configured to insert the device 1 for example for a few meters, whereafter the device 1 begins to propulse into the soil 2 using its movement elements 10, thereby investigating the soil 2.

The deployment unit 21 can comprise a control unit configured to send output signals to the device 1 and to receive input signals from the device 1. For example, the output signals sent to the device 1 relate to and control the movement of the device 1 in the soil 2, such as a maximum depth of soil 2 below seabed 6 to be investigated by the device 1 and/or a steering direction of the device 1 as it propulses in the soil 2. The input signals can comprise data concerning a determined quantity of at least one physical parameter which is indicative of a force acting on the respective movement element 10 when the respective movement element 10 moves in the soil 2. For example, the data relate to force values representing the driving force of an actuator 15 of the device 1 required to move a movement element 10 against the soil 2, which represents the counterforce exerted by the soil 2 on the movement element 10, and optionally the corresponding displacement, thereby allowing to quantify the properties of the soil 2. To exchange the input and the output signals between the device 1 and the deployment unit 21, the device 1 and the deployment unit 21 may communicate via a cable connection or a wireless connection.

The deployment unit 21 can comprise an energy supply unit configured to supply the device 1 with energy required for the propulsion of the device 1 by means of its movement elements 10. For example, the energy supply unit comprises a hydraulic system with a hydraulic liquid reservoir and a hydraulic pump for realizing the movement of the movement elements 10 by means of hydraulic actuators of the device 1. To this end, the deployment unit 21 and its hydraulic system can be connected by hydraulic tubes for conducting the hydraulic liquid to the hydraulic actuators of the device 1 that realize the movement of the movement elements 10.

The watercraft 30 may deploy multiple systems 20 for simultaneously investigating soil 2 at different offshore locations, and indicate their locations using buoys 32, as indicated in section (g) Fig. 4. As such, the invention may be for example applied to investigate soil properties at sites and evaluate the suitability of possible sites for offshore installations, for example for wind energy infrastructure.

Upon the investigation of the soil 2, the device 1 is retrieved by the deployment unit 21, cf. section (h), (i) and (j) in Fig. 4. To this end, the device 1 can move back to the seabed 6 using its movement elements 10 or the deployment unit 21 pulls the device 1 back to the seabed 6, for example by winding a cord connecting the device 1 and the deployment unit 21 and/or by winding the cable for the communication between the device 1 and the deployment unit 21.

**List of reference signs**

| | |
|---|---|
| Device | 1 |
| Soil | 2 |
| Screw conveyor | 3 |
| Hollow cylinder | 4 |
| End face | 5 |
| Seabed | 6 |
| Water reservoir | 7 |
| Movement element | 10 |
| First radial movement device | 11 |
| Second radial movement device | 12 |
| Radial movement element | 13 |
| First radial movement element | 13a |
| Second radial movement element | 13b |
| Axial movement device | 14 |
| Actuator | 15 |
| Shear wave emitter | 16a |
| Shear wave detector | 16b |
| Outer surface of radial movement element | 17 |
| Rotary actuator | 18 |
| System | 20 |
| Deployment unit | 21 |
| Frame | 22 |
| Watercraft | 30 |
| Cutting edge | 31 |
| Buoy | 32 |
| First axial movement element | 41 |
| Second axial movement element | 42 |
| Central section | 43 |
| First end face | 51 |
| Second end face | 52 |
| Inner surface of second end face | 52a |
| Longitudinal axis | L |

## Claims

1. A device (1) for investigating soil (2), comprising movement elements (10) for propulsion of the device (1) in the soil (2), wherein the device (1) is configured to determine a quantity of at least one physical parameter which is indicative of a force acting on at least one of the movement elements (10) when the respective movement element (10) moves in the soil (2).

2. The device (1) for investigating soil (2) according to claim 1, wherein the device (1) is configured to generate a driving force for causing the movement of the respective movement element (10) in the soil (2), wherein the physical parameter is the driving force acting on the respective movement element (10).

3. The device (1) for investigating soil (2) according to claim 2, comprising two axial movement elements (41,42) configured to be moved with respect to each other along a longitudinal axis (L) of the device (1).

4. The device (1) for investigating soil (2) according to claim 3, comprising a first radial movement device (11) and a second radial movement device (12), wherein the first radial movement device (11) is arranged at one of the axial movement elements (41) and wherein the second radial movement device (12) is arranged at the other of the two axial movement elements (42), such that the first radial movement device (11) and the second radial movement device (12) can be moved with respect to each other.

5. The device (1) for investigating soil (2) according to claim 3 or 4, comprising an axial movement device (14) for extending the device (1) along its longitudinal axis (L) by increasing an offset between the two axial movement elements (41,42).

6. The device (1) for investigating soil (2) according to one of the claims 3 to 5, wherein the first radial movement device (11) and the second radial movement device (12) each comprise at least one radial movement element (13) configured to be moved radially outwards against the soil (2) by means of a respective driving force, wherein the movement of at least one of the radial movement elements (13) and/or the axial movement elements (41,42) is configured to be at least partially reversed.

7. The device (1) for investigating soil according to one of the preceding claims, comprising a shear wave emitter (16a) and a shear wave detector (16b), wherein the shear wave emitter (16a) is configured to emit a shear wave into the soil and wherein the shear wave detector (16b) is configured to detect the arrival of the shear wave travelling through the soil.

8. The device (1) for investigating soil (2) according to one of the preceding claims, comprising a screw conveyor (3) extending along a longitudinal axis (L) of the device (1), and wherein the device (1) comprises a rotary actuator for causing a rotational motion of the screw conveyor (3) around the longitudinal axis (L), such that soil (2) can be conveyed by the screw conveyor (3) and that the device (1) can be moved through the soil (2) under rotation of the screw conveyor (3).

9. The device (1) for investigating soil (2) according to one of the claims 4 to 8, wherein the first radial movement device (11) comprises a plurality of first radial movement elements (13a), for instance four first radial movement elements (13a), and/or wherein the second radial movement device (12) comprises a plurality of second radial movement elements (13b), for instance four second radial movement elements (13b), wherein at least some of the radial movement elements (13a, 13b) are arranged equidistantly around the longitudinal axis (L).

10. The device (1) for investigating soil (2) according to one of the preceding claims, wherein at least one of the movement elements (10,13,13a,13b,41,42) is mechanically connected to an actuator (15) for carrying out the movement of the at least one movement element (10,13,13a,13b,41,42) in the soil (2).

11. The device (1) for investigating soil (2) according to claim 10, wherein the actuator is a hydraulic actuator and the driving force for causing the movement of the respective movement element (10) in the soil (2) depends on a hydraulic pressure generated by the hydraulic actuator, and wherein the device further comprises a hydraulic pressure sensor configured to determine the hydraulic pressure generated by the hydraulic actuator as the physical parameter.

12. The device (1) for investigating soil (2) according to one of the preceding claims, wherein the device (1) is configured to determine a force, a displacement and/or a time associated with the movement of the at least one movement element (10,13,13a,13b,41,42) in the soil (2).

13. A system (20) for investigating soil (2), comprising at least one device (1) for investigating soil (2) according to one of the claims 1 to 12, as well as a deployment unit (21) for positioning the device (1) on a soil surface, such that soil (2) below the soil surface can be investigated using the device (1).

14. A method for investigating soil (2), wherein at least one device (1) for investigating soil (2) according to one of the claims 1 to 12 or at least one system (20) for investigating soil (2) according to claim 13 is positioned on soil (2) and wherein movement elements (10,13,13a,13b,41,42) of the device (1) of the system (20) propulse the device (1) in the soil (2) and at least one quantity of a physical parameter which is indicative of a force acting on at least one of the movement elements (10) when the respective movement element (10,13,13a,13b,41,42) moves in the soil (2) is determined.

15. The method according to claim 14, wherein the device (1) for investigating soil (2) according to one of the claims 1 to 12 or the system (10) is positioned on the soil surface and the device (1) inserts or is inserted into the soil (2), and wherein soil (2) below the soil surface is investigated using the device (1).
